# EUROPEAN PATENT APPLICATION

(11) **EP 3 323 510 A1**
(43) Date of publication of application: **23.05.2018**
(21) Application number: 16199852.1
(22) Date of filing: 21.11.2016
(51) Int. Cl.: B01J 37/08, B01J 37/34, B01J 23/00, B01J 23/887, B01J 35/00, C01B 13/18, C07C 45/35, C07C 51/25, B01J 35/10, B01J 37/00, C01G 39/00

(54) **PROCESS FOR THE PREPARATION OF A MIXED OXIDE CATALYST BY FLAME SPRAY PYROLYSIS**

(71) Applicant: Evonik Degussa GmbH, 45128 Essen (DE)
(72) Inventor: SPRENGER, Paul, 76131 Karlsruhe (DE); GRUNWALDT, Jan-Dierk, 76297 Stutensee (DE); KLEIST, Wolfgang, 76149 Karlsruhe (DE); FISCHER, Achim, 63773 Goldbach (DE)

(57) **Abstract**

The process of the present invention relates to a process for the preparation of a mixed oxide catalyst, comprising bismuth, cobalt, iron and molybdenum, comprising the steps a) providing a solution, suspension and/or slurry comprising precursors of bismuth, cobalt, iron and molybdenum in an organic solvent, b) converting the solution, suspension and/or slurry of step a) to an aerosol, c) introducing the aerosol of step b) into a directly or indirectly heated pyrolysis zone, d) performing a a flame spray pyrolysis to give mixed oxide particles, and e) collecting the mixed oxide particles obtained in step d).

## Description

The present invention relates to a process for the preparation of a mixed oxide catalyst via flame spray pyrolysis, a flame sprayed mixed oxide catalyst and the use of this mixed oxide catalyst in the oxidation of propylene.

Mixed oxides on the basis of bismuth and molybdenum are industrially used as catalysts in the preparation of bulk chemicals such as acrolein or methacrolein as well as acrylic acid and methacrylic acid. Acrolein is amongst others an important starting material for the chemical production of the essential amino acid methionine. Acrylic acid is an important starting material for the production of plastics such as Plexiglas^{®}. A number of different preparation methods, such as co-precipitation, hydrothermal synthesis, and spray drying, each under different preparation conditions, have been applied in the preparation of mixed oxide catalysts based on bismuth and molybdenum. However, all of these preparation methods are either complicated or limited in their application possibilities. The co-precipitation is a rather complicated preparation method because it involves several preparation steps and each of them has a significant influence on the characteristics and the performance quality of the thus prepared catalyst. For example, the procedure of providing the solutions from which the co-precipitation is performed, significantly influences the catalytic performance. In the hydrothermal synthesis the pH values have a big influence on the surface areas and phases, and thus also on the catalytic activity of the hydrothermally prepared catalysts. In spray drying the temperature has a big influence on the size of the particles being formed during the process and also on the particle size distribution, which has a big influence on the catalytic performance.

In addition to the catalytic activity and the specific product selectivity of mixed oxides catalysts the thermal stability of the catalysts, when used in catalytic reaction, is another important factor. In particular, the high temperatures in the hot spots in the catalyst bed during the oxidation of propylene lead to an aging of the used bismuth and molybdenum based catalysts. It is believed that the water vapor, which is always present in the catalyzed oxidation of propylene, either being introduced into the reaction with the feed gas or being formed during the oxidation of propylene, reacts with the oxidic molybdenum of the mixed oxide catalyst under formation of the highly volatile MoO₂(OH)₂. Furthermore, it is believed, that said highly volatile MoO₂(OH)₂ sublimates continuously from the mixed oxide catalyst in the catalyst fixed bed during the performance of the catalyzed reaction. This effect is particularly strong within the so-called hot spots of the reaction or of the catalyst fixed bed, where the reaction heat released in the reaction is the highest. After passing one or more hot spots the temperature drops from its maximum value to a lower temperature, which allows the MoO₂(OH)₂ to condense at the inner wall of the reaction tube. After the catalyst is depleted on the catalytically active element molybdenum, the hot spot moves along the catalyst fixed bed in the direction of flow of the gases towards areas, where the temperature was lower before and which thus are not depleted on molybdenum. However, these areas also deplete on molybdenum continuously. Due to the band-wise depletion of the mixed oxide catalyst on molybdenum, this process is also called band aging. With increasing operating time not only the amount of molybdenum in the catalyst decreases but more importantly, the activity of the catalyst for the oxidation of propylene decreases. This effect is visualized through the simultaneously decreasing conversion rate of propylene. One could try to compensate for said decreased conversion rate by increasing the temperature of the salt bath, which is placed around the reaction tubes to permit heat transfer into the catalyst fixed bed and from the catalyst fixed bed. In this case the temperature in the catalyst fixed bed, which is typically monitored by means of thermocouples, is increased to values which provide for the same specific conversion of the same starting compound under otherwise unchanged reaction conditions before the decrease of catalyst activity. However, the increase of the temperature of the oven or salt bath around the reaction tubes or of the catalyst fixed bed in order to counteract on the decreased catalyst activity promotes the further sublimation of molybdenum from the mixed oxide catalyst. In addition, this further loss of molybdenum is no longer limited to the areas of the hot spots, but is more or less extended over the whole length at which mixed oxide catalyst is present in the catalyst fixed bed. Accordingly, said procedure is contra-productive and leads to an early deterioration of the complete mixed oxide catalyst, independently from the previous position of the hot spot(s). In any case, said depletion of the mixed oxide catalyst on molybdenum makes it necessary to replace at least parts of the exhausted catalyst fixed with new catalyst. This leads to plant downtimes and losses of production.

It is therefore advantageous to use mixed oxide catalysts in the oxidation of propylene, which lease a heat tone as low as possible during the catalyzed oxidation of propylene in particular in the hot spots of the catalyst fixed bed. However, the specific preparation of such catalysts is still a big challenge. The choice of the elements is severely limited by the necessity of the catalytically active elements. Thus, only the variation of the amounts of said catalytically active elements and optionally, the presence of additional elements can influence the reaction heat, which is released during the catalyzed oxidation of propylene. However, it is not understood yet, how the reaction heat in the catalyzed oxidation of propylene can be specifically influence by varying the amounts of said catalytically active elements and the optional presence of additional elements. Thus, the provision of mixed oxide catalysts which give a lower reaction heat in the oxidation of propylene is still a matter of luck.

Thus, there is a need for an uncomplicated process for the preparation of improved mixed oxide catalysts for the use in the preparation of unsaturated aldehydes and carboxylic as well as unsaturated nitriles.

It was found that flame spray pyrolysis permit the preparation of improved mixed metal oxides for the preparation of unsaturated aldehydes and carboxylic as well as unsaturated nitriles.

One object of the present invention is therefore a process for the preparation of a mixed oxide catalyst, comprising bismuth, cobalt, iron and molybdenum, comprising the steps
a) providing a solution, suspension and/or slurry comprising precursors of bismuth, cobalt, iron and molybdenum in an organic solvent,
b) converting the solution, suspension and/or slurry of step a) to an aerosol,
c) introducing the aerosol of step b) into a pyrolysis zone,
d) performing a flame spray pyrolysis to give mixed oxide particles, and
e) collecting the mixed oxide particles obtained in step d).

In the context of the present invention the term flame spray pyrolysis is used to denote any process in which the metal precursors are decomposed by burning the precursors in one or more flames and the thus obtained metals are subsequently oxidized to give the metal oxides of the mixed metal oxide through the flame action.

In the process according to the present invention the metal comprising precursors are introduced as an aerosol to the pyrolysis zone. Said aerosol is preferably obtained by nebulization of the solution, suspension and/or slurry comprising precursors of bismuth, cobalt, iron, and molybdenum in an organic solvent with a carrier gas. Regarding the choice of the carrier gas the process of the present invention is not subject to any limitation, provided that said carrier gas provides for a sufficient nebulization of the precursors. Notwithstanding, said carrier gas advantageously supports the formation of the mixed oxide particles. Therefore, a preferred carrier gas is air, oxygen, air enriched with oxygen or a mixture thereof.

For nebulization the solution, suspension and/or slurry is typically first placed in a syringe, feed to a nozzle by means of a syringe pump, released through a capillary, dispersed with the carrier gas and pressed through the nozzle under formation of the aerosol into the pyrolysis zone. In this case, the conversion of the solution, suspension and/or slurry to the aerosol and the introduction of said aerosol into the pyrolysis zone are carried out at the same time. Alternatively, it is also possible, to provide the aerosol first, followed by introduction of the aerosol into the pyrolysis zone. Regarding the number of nozzles, the process according to the present invention is not subject to any limitation, rather the respective apparatus for the preparation of the mixed oxide catalysts by pyrolysis can have one or more nozzles. In case said apparatus has more than one nozzle, the solution, suspension and/or slurry with the same composition or different compositions of the respective elements are fed to the different nozzles, in order to provide for a homogeneous distribution of the elements in the mixed oxide particle to be prepared.

In the pyrolysis zone the nebulized precursor are decomposed to give a vapor, which is believed to consist of gaseous metal ions and oxyanion species. Said vapor is oxidized and the thus obtained metal oxide species are believed to nucleate and grow to form clusters of metal oxide bonds. These clusters condense to form nuclei, which subsequently grow by consuming the vapor phase species and bonding with oxygen available in the atmosphere. In this context, the term cluster refers to the initially generated species that form as a vapor. These clusters coalesce to form nuclei, which later form stable particles. Once formed, nuclei collide to coalesce or agglomerate, where the mechanisms is dictated by the temperature and the individual species, for example cooling changes the effect of collision from coalescence to agglomeration.

Typically, the temperature in the pyrolysis zone is above the decomposition temperature of the precursor and is sufficiently high for the formation of oxides of the respective metals. Preferably, the temperature at which the pyrolysis is performed ranges from 500 to 2000°C, especially from 900 to 1500°C, irrespective of whether the pyrolysis zone is heated directly or indirectly.

In the context of the present invention it was found that the flame spray pyrolysis is suitable for providing improved mixed oxide catalysts, comprising bismuth, cobalt, iron and molybdenum, which give a high acrolein selectivity at a significantly higher propylene conversion than the flame-sprayed bismuth molydate catalysts of the prior art and the co-precipitated mixed oxide catalysts of the prior art. For example, the flame sprayed catalyst with the composition Bi_{1.5}Mo₁₂Co₈Fe_{1.8}Ox gives an acrolein selectivity of up to 100 % at a w/F_{feed} = 0.24 g s ml⁻¹; by comparison, the co-precipitated catalyst having the same composition only gives an acrolein selectivity of 79 % at the same w/F_{feed} ratio and there was no propylene conversion activity and no acrolein selectivity detectable for the flame sprayed bismuth molybdate catalysts with the compositions Bi₂Mo₂O₉ and Bi₂MoO₆ of the prior art (K. Schuh, W. Kleist, M. Høy, V. Trouillet, A. D. Jensen and J.-D. Grunwaldt, Chem. Commun., 2014, 50, 15404-15406).

Comparative tests showed that said bismuth molybdate catalysts of the prior art, and in particular said Bi₂Mo₂O₉ catalyst, have a significantly lower activity than the catalysts obtained by the process according to the present invention, even at higher temperatures and at a higher ratio of catalyst mass to feed flow (w_{cat}/F_{feed}). However, catalysts with such a low propylene conversion are not suitable for any industrial application since large volumes of unreacted starting material had to be fed back into the process. This would require a larger reactor volume, and a significantly higher energy input for the condensation and the recycling of the unreacted starting material.

In addition, due to the significantly higher activity of the catalysts obtained by the process according to the present invention, a lower temperature is required for the heat transfer medium surrounding the catalyst fixed bed. This has the benefit that a lower energy input is required to achieve a specific propylene conversion, which results in a large energy saving. A further benefit of the lower energy input is that the temperature within the catalyst fixed bed is also lowered. For example, when the oven temperature for testing the flame sprayed catalysts obtained by the process according to the present invention is 320°C, a temperature of approximately 360°C is measured in the catalyst fixed bed. By comparison, a temperature of approximately 380°C is measured in the catalyst fixed bed of the reference catalysts at an oven temperature of 362°C.

This does not only lead to a reduced energy input in the production of acrolein in the presence of the catalysts obtained by the process according to the present invention but also to a reduced thermic stress of the catalysts, which also has a positive effect on the lifetime of the catalysts of the present invention in the catalyzed production of acrolein.

A further advantage of preparing mixed oxide catalysts by flame spray pyrolysis is that approximately the complete metal fractions provided in the solution, suspension or slurry comprising the metal precursors are incorporated into the final mixed oxide catalyst. This has the benefit that the catalysts with the desired composition can be very accurately and reproducibly be prepared. By comparison, other techniques for preparing mixed oxide catalysts, such as co-precipitation, do not always lead to the desired composition since it cannot be ruled that the complete metal fractions provided in a solution, suspension or slurry are incorporated into the final catalysts. It is therefore another benefit that the process according to the present invention makes very efficient use of the materials, which are used in this process. Considering the rare availability of some catalytically active elements and their high prices, the process according to the present invention makes a big contribution with respect to sustainability.

In the process according to the present invention the pyrolysis zone is a flame spray pyrolysis. Typically, the flame spray pyrolysis is achieved by burning the metal precursors in one or more flames. In this case, the pyrolysis zone also contains an ignition device. For this type of direct heating, combustible gases are usually used for providing the flame. Regarding the choice of the combustible gas, the flame spray pyrolysis according to an embodiment of the process of the present invention is not subject to any limitation, provided that said combustible gas can provide a flame with a temperature, which is sufficiently for carrying out the pyrolysis. In context of the present invention hydrogen, methane, ethane and mixtures thereof are preferred combustible gases for the flame spray pyrolysis. The temperature in the pyrolysis zone can be adjusted as required or as desired by means of the ratio of the amount of combustible gas to the total amount of gas used for providing the aerosol. In the case of a flame spray pyrolysis the carrier gas, used for the nebulization of the solution, suspension and/or slurry comprising precursors of bismuth, cobalt, iron, and molybdenum in an organic solvent, is preferably a gas, which supports the burning of the flame and the formation of the mixed oxide particles in the pyrolysis zone. A preferred carrier gas is therefore air, oxygen, air enriched with oxygen or a mixture thereof.

According to the process of the present invention the mixed oxide particles generated in the pyrolysis are collected. It was found that very small particles of nanoparticle size are obtained when the mixed oxide particles obtained in the flame spray pyrolysis are quenched on a cold surface.

In one embodiment of the process according to the present invention the step e) of collecting the mixed oxide particles obtained in step d) is performed by quenching of the mixed oxide particles on a cold surface.

Preferably, the mixed oxide particles obtained from the pyrolysis are collected by means of a filter, which is preferably connected to a vacuum pump. Therefore, the quenching is preferably a rapid quenching. Preferably, said filter has a temperature of not more than 100°C. This permits a rapid quenching of the mixed oxide particles. Thus, in fractions of seconds the metal precursors are heated from room temperature to the temperatures at which the flame pyrolysis is performed, i.e. temperatures of from 500 to 2000°C, preferably from 900 to 1500°C, and again in fractions of second the mixed oxide particles obtained in the flame spray pyrolysis are cooled to room temperatures. This has the advantage, that a sintering of the mixed oxide particles is avoided, which favors the collection of very small nano particles. Thus, a powder is obtained whose very high surface area is based on its crystallite size, in contrast to the typically obtained mixed oxide catalysts whose surface area is due to their porosity. A coking during the catalyzed reaction, however, can lead to a blocking of the pores of the catalysts of the prior art, whose activity is thus significantly reduced. By comparison, the activity of the mixed oxide catalysts obtained by the process according to the present invention is much less negatively influenced by coking.

Preferably, the mixed oxide particles formed in the pyrolysis are collected at a height of 20 to 80 cm, preferably at a height of 40 to 60 cm, over the pyrolysis zone, preferably over the flame of the flame spray pyrolysis, when an amount of 10 to 20 g of mixed oxide particles per hour is produced.

The process according to the present invention involves the use of an organic solvent, i.e. a carbon and hydrogen containing compound, rather than water as solvent for the provision of a solution, suspension and/or slurry of the starting materials. This has the advantage that the organic compound itself used as solvent supports the burning of the flame in the flame spray pyrolysis because of its burnable components carbon and hydrogen. A further advantage of using an organic compound as solvent instead of a water is that the complete combustion heat is used for the maintenance of the flame and the combustion but not for the evaporation of the water. In the context of the present invention said organic solvent is preferably a carbon rich compound which provides a calorific value as high as possible for supporting the flame in the flame spray pyrolysis. This has the further advantage of a high burning temperature, which is beneficial for the performance of the flame spray pyrolysis, in particular for a conversion of the bismuth, cobalt, iron and molybdenum in the starting materials to the corresponding oxide in the mixed oxide catalyst as completely as possible. In addition, the composition can be easily varied and further dopants can be introduced. Regarding the choice of the organic solvent the process according to the present invention is not subject to any limitation, provided that the precursors of bismuth, cobalt, iron and molybdenum are soluble in the respective organic solvent.

Suitable carbon rich organic solvents in the context of the present invention are liquids at room temperature, so that they can easily be transformed into an aerosol with a suitable carrier gas, in particular with any of the preferred carrier gases. Preferred organic compounds for use as organic solvent in the context of the present invention are hydrocarbons with 5 to 15 carbon atoms, for example toluene, xylene, n-heptane, n-pentane, octane, iso-octane, cyclohexane, methyl, ethyl or butyl acetates or mixtures thereof. A preferred organic solvent in the context of the present invention is xylene, since its use permits the provision of solutions of a wide variety of bismuth, cobalt, iron and molybdenum comprising precursors.

Regarding the choice of the bismuth, cobalt, iron and molybdenum comprising precursor the process according to the present invention is not subject to any limitations, provided that the precursors are sufficiently soluble, dispersible and/or suspendible in the chosen organic solvent. In order to provide for an aerosol as good as possible, it is preferred to provide a solution of bismuth, cobalt, iron, and molybdenum comprising precursors in an organic solvent. It is further preferred to use a solution which contains all the relevant precursors, in order to provide for a homogeneous distribution of the precursors and the metals in the aerosol. It is therefore advantageous to use organic compounds or bismuth, cobalt, iron and molybdenum as precursors.

In one embodiment of the process according to the present invention the precursors are therefore organic compounds of bismuth, cobalt, iron and molybdenum.

Suitable organic compounds of bismuth, cobalt, iron and molybdenum are salts and/or complexed of bismuth, cobalt, iron and molybdenum with an organic acid, an organic alcohol and/or a chelating agent because they are easily soluble in organic solvents, and in particular, in hydrocarbons with 5 to 15 carbon atoms, for example toluene, xylene, n-heptane, n-pentane, octane, iso-octane, cyclohexane, methyl, ethyl or butyl acetates or mixtures thereof.

In another embodiment of the process according to the present invention the precursors are therefore salts and/or complexes of bismuth, molybdenum, cobalt and iron with an organic acid, organic alcohol and/or a chelating agent.

In order to provide for homogeneous distribution of the bismuth, molybdenum, cobalt and iron comprising precursors in the organic solvent, the organic acid, organic and/or chelating agent of said precursor has a similar polarity as the organic solvent, in particular a similar number of carbon atoms. Suitable organic acids for forming a salt with bismuth, cobalt, iron and/or molybdenum are acetic acid, propionic acid, oxalic acid, octanoic acid, 2-ethylhexanoic acid, and neodecanoic acid. Suitable organic alcohols for forming a salt with bismuth, cobalt, iron and/or molybdenum are ethanol, n-propanol, iso-propanol, n-butanol and tert-butanol. Particularly suitable for forming the salts are organic acids, especially the aforementioned organic acids.

In a preferred embodiment of the process according to the present invention the organic acid is a linear or branched C₁ to C₁₀ carboxylic acid.

In the context of the present invention 2-ethylhexanoic acid has be proven to be very suitable for forming a salt with bismuth, cobalt, iron and/or molybdenum.

In another preferred embodiment of the process according to the present invention the organic acid is 2-ethylhexanoic acid.

Regarding the choice of the chelating agent for forming a complex with bismuth, cobalt, iron and/or molybdenum, the requirements are the same as for the formation of a salt, provided that the chelating agent can form a complex with a metal of a specific charge.

In a preferred embodiment of the process according to the present invention the chelating agent is therefore a C₁ to C₁₀ compound with a hydroxyl, carbonyl, amino and/or amide group. Preferably, the chelating agent has one or two hydroxyl, carbonyl, amino and/or amide groups.

In the context of the present invention acetylaceton has be proven to be very suitable for forming a complex with bismuth, cobalt, iron and/or molybdenum.

In another preferred embodiment of the process according to the present invention the chelating agent is acetylaceton.

In particular, the bismuth comprising precursor is bismuth 2-ethylhexanoate, preferably bismuth(III) 2-ethylhexanoate. In particular, the molybdenum comprising precursor is molybdenum 2-ethlhexanoate, preferably molybdenum(IV) 2-ethylhexanoate. In particular, the cobalt comprising precursor is cobalt 2-ethylhexanoate, preferably cobalt(II) 2-ethylhexanoate. In particular, the iron comprising precursor is iron acetylacetonate, preferably iron(III) acetylacetonate.

Even though, the mixed oxide particles are generated through pyrolysis, it is not notwithstanding preferred to subject them to a drying at temperatures of more than 100°C in order to free the particles from any traces of water or any other compounds, for example organic compounds which could be the result from condensation during or after the collection of the particles. Preferably, the drying is performed for a period of at least 1 hour, especially the drying period ranges from 2 to 6 hours. Preferably, the temperature of the drying ranges from 200 to 400°C.

The process according to the present invention provides the mixed oxide catalysts typically in powder form. However, for an industrial application it is preferred to apply the powdery mixed oxide catalyst onto a carrier. Suitable carriers for the mixed oxide catalysts obtained by the process according to the present invention are for example aluminum oxide, titanium dioxide, silicon dioxide and zirconium dioxide. In principle, the process according to the present invention is not subject to any limitations regarding the amount of nozzles in the flame spray pyrolysis. It is therefore possible to perform the flame spraying of the present invention with one, two, three, four or five or even more nozzles through which the solution, suspension or slurry with the metal precursors are nebulized to provide the respective aerosol(s) and to nebulize a solution with a precursor of the support material through one or more further nozzles.

In an embodiment the process according to the present invention further comprises providing a solution, suspension and/or slurry comprising a precursor of a carrier material in step a), converting the same to an aerosol in step b), introducing the aerosol into the pyrolysis zone in step c), performing a flame spray pyrolysis to give supported mixed oxide particles in step d), and collecting the supported mixed oxide particles in step e).

In said pyrolysis zone not only the precursors of bismuth, cobalt, iron and molybdenum but also the precursors of the carrier are converted to the respective oxides. Suitable precursors of a carrier material are in principle organic compounds of the same types as in the case of the precursors of bismuth, cobalt, iron and molybdenum. Preferably, the silicon comprising precursor for silicon dioxide as carrier material is hexamethyldisiloxane, which is preferably dissolved in 2-ethylhexanoic acid, methanol or acetic acid or a mixture thereof. Preferably, the aluminum comprising precursor for aluminum oxide is aluminum acetylacetonate, which is dissolved in methanol or acetic acid or a mixture thereof. Preferably, the titanium comprising precursor for titanium dioxide is titanium tetraiso-propoxide, which is dissolved in propionic acid, 2-ethylhexanoic acid or methanol or a mixture thereof. Due to the higher polarity of these precursors xylene is no longer a suitable organic solvent and has to be replaced with more polar organic solvents, such as those mentioned before.

Notwithstanding that the mixed oxide particles obtained in the process according to the present invention are directly applied onto the in situ produced carrier material, it is also possible to apply the thus obtained supported mixed oxide particles onto a carrier in a further step, into to provide for larger catalyst particles.

The process according to the present invention provides the mixed oxide catalysts typically in powder form. However, for an industrial application it is preferred to bring the powdery mixed oxide catalyst after addition of a forming and binding agents into a more practicable form. This can be achieved by either applying the mixed oxide catalyst to a carrier, for example a carrier of aluminum oxide, zirconium oxide, titanium dioxide or silicon dioxide, or shaping steps such tableting or extrusion. The geometric shape of the carrier is not subject to any limitations but it rather follows the characteristics of the reactor, for example the inner diameter of the reaction tube, its length and the length of the catalyst filling. The carrier can be for example a pyramid, a cylinder, a saddle, a sphere or a polyhedron, but it can be also the inner wall or a reaction room, for example in form of a wall reactor catalyst.

Suitable binding agents, that can be used in the context of the present invention, are diverse oils, celluloses, polyvinyl alcohols, saccharide, acrylates, alkyl derives thereof, condensates thereof or mixtures thereof. Preferred binding agents are acrylates, polyvinyl alcohols, celluloses, alkyl derivatives thereof and mixtures thereof.

A supported catalyst is preferably prepared by spraying either a suspension of the catalyst in a suitable solvent or solvent mixture together with the binding agent onto the carrier or by spraying a suspension of the catalyst onto a carrier, which has been wetted with the binding agent. Suitable solvent are for example water, methanol, ethanol, or similar compounds of mixture thereof. When particles of a carrier are used, the loaded particles can also be also mixed with a binding agent and then be subjected to a tableting procedure. A solid catalyst can be prepared by first mixing the catalyst with a suitable binding agent as mentioned before, followed by shaping.

In the following, the catalyst obtained by the individual shaping procedure is subjected to a drying and/or calcining for a completion of the removal of any solvents and the binding agent.

In another embodiment the process according to the present invention further comprises the steps:
f) applying the mixed oxide particles or supported mixed oxide particles obtained in step e) to a carrier to yield a supported catalyst,
   or
f') shaping the mixed oxide particles or supported mixed oxide particles obtained in step e) to yield a solid catalyst,
   and
g) drying and/or calcining the supported or solid catalyst obtained in step f) or f').

Preferably, the drying of the supported catalyst and/or of the solid catalyst is performed at a temperature of more than 100°C in order to free the particles from any traces of water or any other compounds, for example organic compounds which could be the result from condensation during or after the steps f) and/or f'). Preferably, the drying is performed for a period of at least 1 hour, especially the drying period ranges from 2 to 6 hours. Preferably, the temperature of the drying ranges from 200 to 400°C. The calcination is preferably carried out at a temperature of more than 400°C, in particular at a temperature of at least 500°C. Preferably, the calcination is carried out for at least 1 hours, in particular for a period of from 2 to 5 hours.

Typical carriers for a mixed oxide catalysts are aluminumoxide, silicon dioxide, titanium dioxide and zirconium dioxide.

According to the literature (see for example K. Schuh, W. Kleist, M. Holy, V. Trouillet, A. D. Jensen and J.-D. Grunwaldt, Chem. Commun., 2014, 50, 15404-15406) the beta-phase of bismuth molybdates, i.e. β-Bi₂Mo₂O₆, gives the highest acrolein selectivity. However, said beta-phase is metastable at temperature below 540°C and leads to its decomposition into α-Bi₂Mo₃O₁₂ and γ-Bi₂MoO₆. Accordingly, said flame-sprayed catalyst is not suitable for an industrial application due to its lack of thermal stability in the range of temperatures, which are typically applied in the oxidation of olefins to give unsaturated aldehydes and/or carboxylic acids or in the ammonoxidation of olefins to give unsaturated nitriles. Any attempt to avoid the decomposition of β-Bi₂Mo₂O₆ by applying temperatures of more than 540°C, however, would result in the loss of the catalytically active component molybdenum, which would react with water to give the highly volatile MoO₂(OH)₂, followed by the sublimation of this derivative from the catalyst in the catalyzed reaction. A further disadvantage of the β-Bi₂Mo₂₆ phase is that it is decomposed at reductive conditions in the catalyzed reaction, for example an excess of the olefin(s) and/or a deficit of oxygen.

By comparison, it is believed that the increased activity in the propylene conversion and the very good acrolein selectivity of the mixed oxide catalysts obtained by the process according to the present invention is the result of the presence of a cobalt-molybdenum phase, an iron-molybdenum phase and/or a mixed phase thereof in the mixed oxide catalysts. The presence of a cobalt-molybdenum phase, an iron-molybdenum phase and a mixed phase thereof has been proven by ex situ Raman spectroscopy and X-ray diffraction patterns of catalyst samples, which were prepared by the process according to the present invention, before and after their use in the catalyzed oxidation of propylene. Thus, in contrast to the flame-sprayed bismuth molybdate catalysts of the prior art, the mixed oxide catalysts of the present invention have catalytically active phases, which are thermally stable. This makes the catalysts of the present invention very attractive for use in catalyzed reactions.

Another object of the present invention is therefore a mixed oxide catalyst, comprising bismuth, cobalt, iron and molybdenum, wherein said catalyst comprises a cobalt-molybdenum phase, an iron-molybdenum phase and/or a mixture of these phases.

In particular, the increased activity in the propylene conversion and the very good selectivity are believed to be the result of a Bi₃(FeO₄)(MoO₄)₂, a Co0.7Fe_{0.3}MoO₄ phase or of the presence of both phases. Thus, the mixed oxide catalyst according to the present invention preferably comprises a Bi₃(FeO₄)(MoO₄)₂ phase and/or a Co_{0.7}Fe_{0.3}MoO₄ phase.

The process according to the present invention has been proven to be very suitable for the provision of bismuth, cobalt, iron and molybdenum comprising mixed oxide catalysts with a cobalt-molybdenum phase, an iron-molybdenum phase and/or a mixed phase, preferably with a Bi₃(FeO₄)(MoO₄)₂ phase and/or a phase Co_{0.7}Fe_{0.3}MoO₄.

In addition to the explicitly mentioned elements bismuth, cobalt, iron and molybdenum, the catalyst according to the present invention can also comprise further elements, depending on the specific application and the specific conditions for which the individual catalyst is intended to be used.

In one embodiment the catalyst according to the present invention corresponds to the general formula

MoₐBi_{b}Co_{c}Fe_{d}NiₑX_{f}X'_{g}X"ₕX'"ᵢX""ⱼOₓ (I)

with
X = W and/or P,
X' = Li, Na, K, Rb, Cs, Mg, Ca, Ba and/or Sr,
X" = Ce, Mn, Cr and/or V,
X"' = Nb, Se, Te, Sm, Gd, La, Y, Pd, Pt, Ru and/or Au,
X"" = Si, Al, Ti and/or Zr,
   and
a = 12,
b = 1 to 4,
c = 4 to 10,
d = 1 to 4,
e = 0 to 4,
f = 0 to 5,
g = 0 to 2,
h = 0 to 5,
i = 0 to 2,
j = 0 to 800,
   and
x = a number, which depends on the valence and amount of the elements different from oxygen.

In a further embodiment the mixed oxide catalysts according to the present invention are obtained and/or obtainable by the process according to the present invention.

In particular, the flame spray pyrolysis has proven to be suitable for the provision of the mixed oxide catalysts according to the present invention.

In another embodiment the mixed oxide catalysts according to the present invention is therefore a flame sprayed mixed oxide catalyst. In particular, the mixed oxide catalyst according to the present invention is obtained and/or obtainable by the process according to the present invention.

Typically, the preparation of bismuth, cobalt, iron and molybdenum comprising mixed oxide catalysts by co-precipitation gives mixed catalysts with a particles diameter of at least 55 µm. For example, the co-precipitation method according to the technical teaching of WO 2007/042369 A1 gives mixed oxide catalysts with a particle diameter of 55 µm. By comparison, the flame spray pyrolysis process according to the present invention gives mixed oxide particles with a particle diameter of not more than 100 nm. Typically, particle diameters of not more than 50 nm are measured for the mixed oxide catalysts before the use in a catalyzed reaction. It is believed that the short residence time of the generated mixed oxide particles in the pyrolysis zone does not permit a further growth of the particles beyond the nano-crystalline size. As a result of this very small crystallites and fine nanoparticles are obtained. It is also observed that the nanoparticles only slightly grow during the use in a catalyzed reaction: After the catalytic testing a particle diameter of the mixed oxide particles of not more than 100 nm is measured. Typically, the particle diameter of the mixed oxide particles according to the present invention is in the range of from 30 to 100 nm after the use in a catalyzed reaction.

In a further embodiment the mixed oxide catalysts of the present invention have a particle diameter of not more than 100 nm. Preferably the mixed oxide catalysts of the present invention have a particle diameter in the range of from 10 nm to 50 nm, in particular in the range of from 10 to 30 nm before the use in a catalyzed reaction. Preferably, the mixed oxide catalysts of the present invention have a particle diameter in the range of from 30 nm to 100 nm after the use in a catalyzed reaction. In particular, the mixed oxide catalyst according to the present invention is a flame-sprayed mixed oxide catalysts with a particle diameter of not more than 100 nm, preferably with a particle diameter in the range of from 10 nm to 50 nm, before the use in a catalyzed reaction. In particular, the mixed oxide catalyst according to the present invention is a flame-sprayed mixed oxide catalysts with a particle diameter of not more than 100 nm, preferably with a particle diameter in the range of from 30 nm to 100 nm, after the use in a catalyzed reaction.

The mixed oxide catalysts obtained by the process according to the present invention as well as the mixed oxide catalysts according to the present invention are suitable for the partial gas phase oxidation and the ammoxidation of olefins and/or alcohols.

In the context of the present invention the terms partial oxidation, partial gas phase oxidation and gas phase partial oxidation are used to denote the reaction of organic compounds under reactive action of oxygen, where the organic compound to be oxidized contains at least one more chemically bond oxygen than before. In contrast to a complete oxidation the partial oxidation, partial gas phase oxidation and gas phase partial oxidation does not lead to an oxidation of all of the carbon atoms in said organic compound.

A further object is therefore the use of a mixed oxide catalyst obtained and/or obtainable by a process according to the present invention and/or of a mixed oxide catalyst according to the present invention in the partial gas phase oxidation and/or ammoxidation of olefins and/or alcohols.

The partial gas phase oxidation of olefins yields unsaturated aldehydes and the corresponding unsaturated carboxylic acid, and the ammoxidation of olefins yields unsaturated nitriles. The economically most relevant unsaturated olefins, carboxylic acids and nitriles are C₃ to C₄ compounds. Therefore, the mixed oxide catalysts obtained by the process according to the present invention and/or the mixed oxide catalyst according to the present invention are preferably used in the catalyzed partial gas phase oxidation of propylene to give acrolein and/or acrylic acid, of iso-buten (2-methyl-1-propen) or tert-butanol (2-methyl-2-propanol) to give methacrolein and/or methacrylic acid, or in the ammoxidation of propylene or iso-buten to give acrylonitrile or methacrylnitrile.

Therefore, in one embodiment of the use according to the present invention the olefin is propylene or iso-buten, and the alcohol is tert-butanol.

The mixed oxide catalysts obtained by a process according to the present invention and the mixed oxide catalysts according to the present invention have proven to be more selective for the formation of unsaturated aldehydes than for the formation of unsaturated carboxylic acids. Therefore, the use according to the present invention is preferably the partial gas phase oxidation of olefins and/or unsaturated alcohols to unsaturated aldehydes. In particular, the use according to the present invention is the partial gas phase oxidation of propylene to acrolein and/or the partial gas phase oxidation of iso-buten and/or ter-butanol to methacrolein.

Another object of the present invention is therefore a method for the preparation of unsaturated aldehydes, unsaturated carboxylic acids and/or unsaturated nitriles, comprising the step of subjecting an olefin and/or an alcohol to a partial gas phase oxidation or to an ammoxidation in the presence of a mixed oxide catalyst according to the present invention and/or in the presence of a mixed oxide catalyst obtained by a process according to the present invention.

Preferably said olefin is propylene, iso-buten (2-methyl-1-propen) and the alcohol is tert-butanol (2-methyl-2-propanol). In that case, the partial gas phase oxidation of propylenes give acrolein and/or acrylic acid, with acrolein as the main product, preferably acrolein as the only product, the partial gas phase oxidation of iso-buten gives methacrolein and/or methacylic acid, with methacrolein as the main product, preferably methacrolein as the only product, and the ammonoxidaton of propylene or iso-buten gives acrylonitrile or methacrylnitrile.

### Description of the Figures:

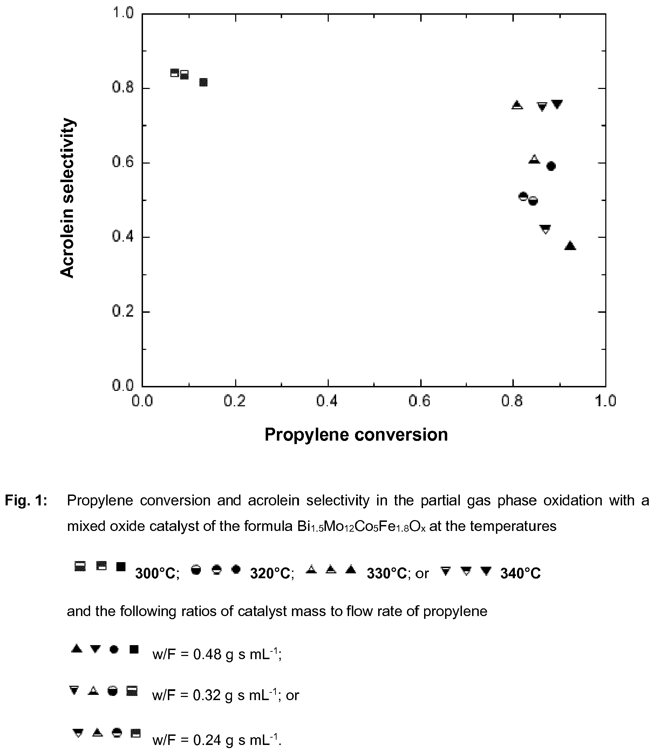

- Fig. 1:: Propylene conversion and acrolein selectivity in the partial gas phase oxidation with a mixed oxide catalyst of the formula Bi_{1.5}Mo₁₂Co₅Fe_{1.8}Oₓ at different temperatures and different ratios of the catalyst mass to the flow rate of the propylene feed.

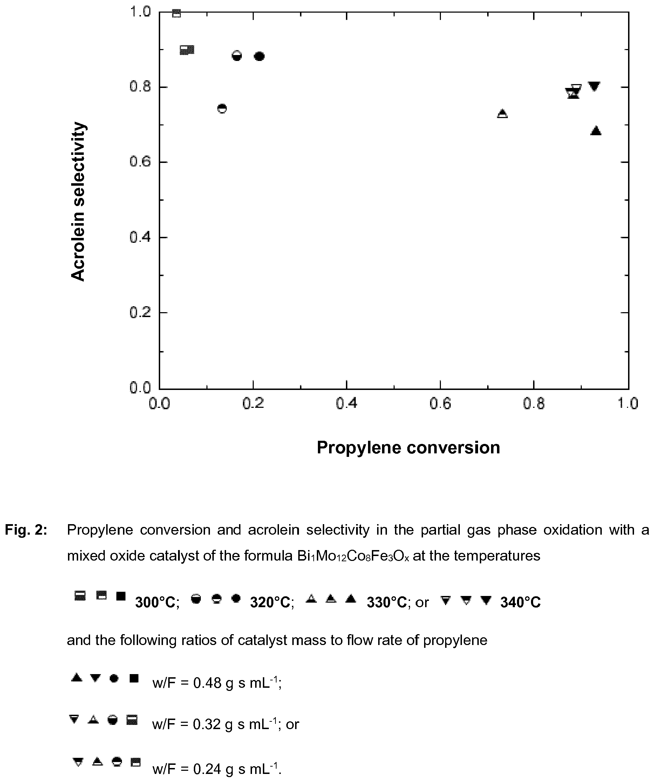

- Fig. 2:: Propylene conversion and acrolein selectivity in the partial gas phase oxidation with a mixed oxide catalyst of the formula Bi₁Mo₁₂Co₈Fe₃Oₓ at different temperatures and different ratios of the catalyst mass to the flow rate of the propylene feed.

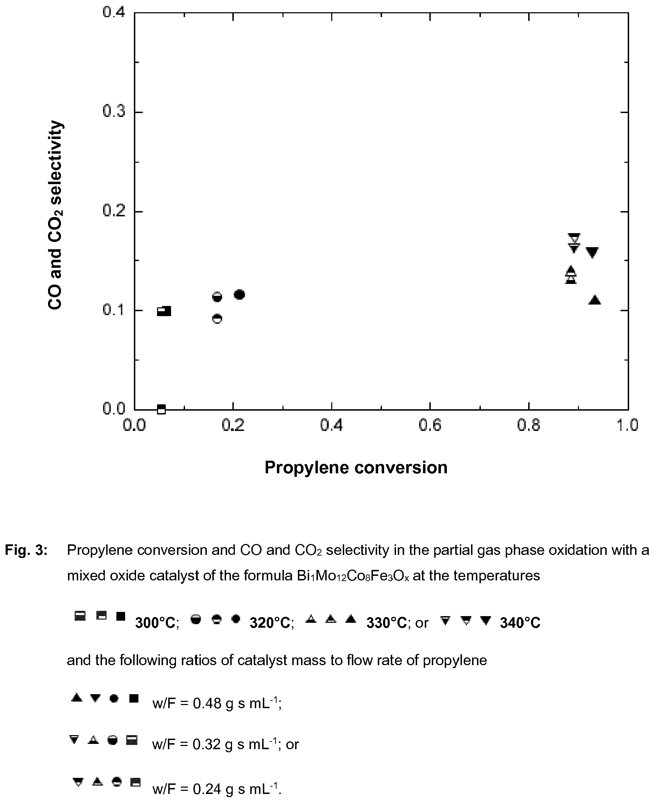

- Fig. 3:: Propylene conversion and CO and CO₂ selectivity in the partial gas phase oxidation with a mixed oxide catalyst of the formula Bi₁Mo₁₂Co₈Fe₃Oₓ at different temperatures and different ratios of the catalyst mass to the flow rate of the propylene feed.

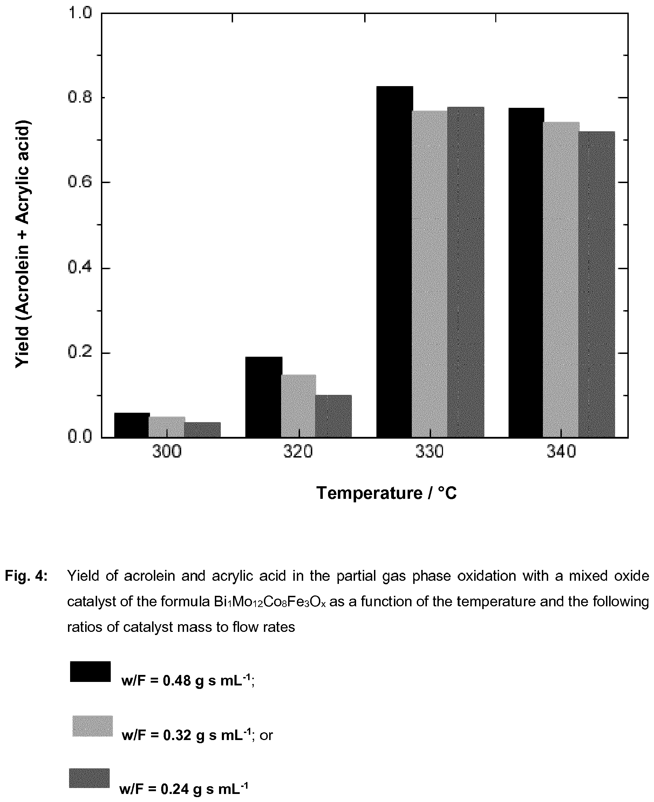

- Fig. 4:: Yield of acrolein and acrylic acid in the partial gas phase oxidation with a mixed oxide catalyst of the formula Bi₁Mo₁₂Co₈Fe₃Oₓ as a function of the temperature and the ratios of catalyst mass to flow rates of propylene feed.
- Fig. 5:: Ex situ Raman spectroscopy of a sample of the flame sprayed catalyst with the composition Bi_{1.5}Mo₁₂Co₅Fe_{1.8}Oₓ, where the lower part is the spectrum of said catalyst after drying at 320°C for 5 hours and the lower part is the spectrum of said catalyst after the catalytic test.
- Fig. 6:: X-ray diffraction pattern of a sample of the flame sprayed catalyst with the composition Bi_{1.5}Mo₁₂Co₅Fe_{1.8}Oₓ before the catalytic test.
- Fig. 7:: X-ray diffraction pattern of a sample of the flame sprayed catalyst with the composition Bi_{1.5}Mo₁₂Co₅Fe_{1.8}Oₓ after the catalytic test.
- Fig. 8:: Optical emission spectroscopy with *i*nductive coupled plasma (ICP-OES) of the mixed oxide catalysts of the examples 1 and 2, wherein the patterned bars indicate the desired composition of the respective catalysts and the plain bars indicate the actual composition of the obtained catalysts, in each case before and after the testing of the respective catalysts.
- Fig. 9:: TEM image of a mixed oxide catalyst with the composition Bi₁Mo₁₂Co₈Fe₃Oₓ before the use in a catalyzed reaction.
- Fig. 10:: TEM image of a mixed oxide catalyst with the composition Bi₁Mo₁₂Co₈Fe₃Oₓ after the use in a catalyzed reaction.

### Examples:

### Example 1: Preparation of a quaternary mixed oxide catalyst

1.144 g of bismuth(III) 2-ethylhexanoate, 9.472 g of molybdenum(IV) 2-ethylhexanoate, 3.297 g of cobalt(II) 2-ethylhexanoate and 0.775 g of iron(III) acetylacetonate were dissolved in 100 mL of xylene to give a total metal concentration of 0.25 M. The resulting solution was filled in syringes having a volume of 50 mL and placed in a syringe pump (World Precision Instruments). The solution was fed to the nozzle of the flame spray pyrolysis with a flow rate of 5 mL/min, dispersed with oxygen having a flow rate of 5 NL/min at a back pressure of 3 bar (back pressure normally varies between 2 and 4 bar), and sprayed through a steel capillary with a diameter of 0.413 (Hamilton syringes, KF6, gauge 22). The dispersed solution was ignited by a supporting flame, obtained through the combustion of methane having a flow rate of 0.75 NL/min with oxygen having a flow rate of 0.6 NL/min. The product particles obtained from the combustion of the solution were collected on a glass fiber filter (Whatman GF6, GE), that was placed 50 cm over the flame and connected to a vacuum pump (R5, Busch). Both, the nozzle and the fiber filter were cooled with water. After dispersion of the 50 mL of the solution in the first syringe into the flame, the fiber filter was replaced with a new fiber and the remaining 50 mL of the solution in the other syringe were dispersed into the flame. Subsequently, the solid powder was collected by scratching it off from the filters with a spatula and sieving it with particle size having a mesh size of 600 µm. Finally, the powder was dried at a temperature of ca. 320°C for a period of 5 hours. The thus obtained mixed oxide catalyst had the formula Bi_{1.5}Mo₁₂Co₅Fe_{1.8}Oₓ.

### Example 2: Preparation of a quaternary mixed oxide catalyst

Example 1 was repeated, this time however, 0.665 g of bismuth(III) 2-ethylhexanoate, 7.995 g of molybdenum(IV) 2-ethylhexanoate, 4.427 g of cobalt(II) 2-ethylhexanoate and 1.104 g of iron(III) acetylacetonate were dissolved in 100 mL of xylene to give a total metal concentration of 0.25 M. The thus obtained mixed oxide catalyst had the formula Bi₁Mo₁₂Co₈Fe₃Oₓ.

### Comparison Example 1: Preparation of a binary mixed oxide catalyst

The procedure of Example 1 was repeated with a solution comprising the starting materials if only two elements. Said solution contained 6.386 g bismuth(III) 2-ethylhexanoate and 9.594 g of molybdenum(IV) 2-ethylhexanoate to give a total metal concentration of 0.25 M. The thus obtained binary oxide catalyst had the formula Bi₂Mo₃O₁₂.

### Comparison Example 2: Preparation of a binary mixed oxide catalyst

The procedure of Example 1 was repeated with a solution comprising the starting materials of only two elements. Said solution contained 7.983 g bismuth(III) 2-ethylhexanoate and 7.995 g of molybdenum(IV) 2-ethylhexanoate to give a total metal concentration of 0.25 M. The thus obtained binary oxide catalyst had the formula Bi₂MoO₆.

### Comparison Example 3: Preparation of a binary mixed oxide catalyst

The procedure of Example 1 was repeated with a solution comprising the starting materials if only two elements. Said solution contained 10.633 g bismuth(III) 2-ethylhexanoate and 5.325 g of molybdenum(IV) 2-ethylhexanoate to give a total metal concentration of 0.25 M. The thus obtained binary oxide catalyst had the formula Bi₂Mo₂O₉.

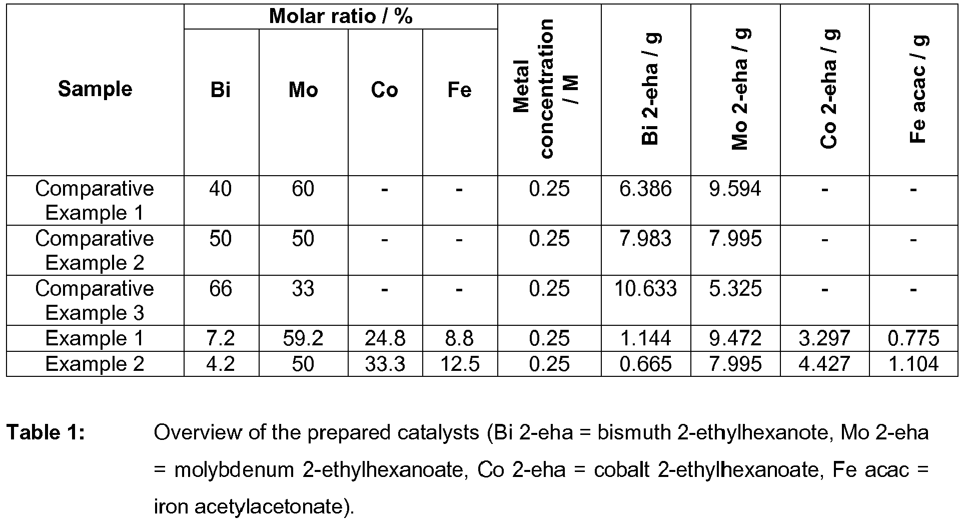

### Example 3: Catalytic testing of the catalyst according to the invention and of comparative catalysts

After drying, the flame sprayed mixed oxide catalyst of example 1 (FSP-Bi_{1.5}Mo₁₂Co₅Fe_{1.8}Oₓ, runs 1 to 4) and the mixed oxide catalyst of example 2 (FSP- Bi₁Mo₁₂Co₈Fe₃Ox, runs 5 to 8) obtained by the process according to the present invention were tested in the partial gas phase oxidation of propylene. In addition, the catalyst with the formula Bi_{1.5}Mo₁₂Co₅Fe_{1.8}Oₓ, which was prepared by the co-precipitation process according to the technical teaching of WO 2007/042369 A1, (CP-Bi_{1.5}Mo₁₂Co₅Fe_{1.8}Oₓ, runs 9 and 10) and the flame sprayed catalysts Bi₂Mo₃O₁₂ (α-phase) (FSP-Bi₂Mo₃0i₂, runs 11 to 13), BbMoO₆ (γ-phase) (FSP- BbMoO₆, runs 14 to 16) and Bi₂Mo₂O₉ (β-phase) (FSP-Bi₂Mo₂O₉, runs 17 to 19) were also tested in the partial gas phase oxidation of propylene. For this purpose a reactor with an inner diameter of 6 mm was filled with a specific mass of the catalyst to be tested having a sieve fraction of from 300 to 450 µm so that the inner volume of the catalyst was completely filled with the catalyst. The resulting catalyst fixed bed had a height of 2.7 cm. Because of the high activity of the catalysts of the examples 3.1 and 3.2, said catalyst was diluted for the catalytic testing: 800 mg of the catalyst were diluted with 800 mg of inert SiC particles having a particle size of from 450 to 600 µm. Because of its lower activity, the 800 mg of the catalyst of the comparative example 3.1 were not diluted. Otherwise, in diluted form the activity of said catalyst would have been too low. Due to their rather low activity, the catalysts of the comparative examples 3.2 and 3.3 were not only used in undiluted form, rather they were even used in the doubled amount of 1600 mg of the respective catalyst. At the upper end and at the lower end of the catalyst was placed a thermocouple, and each of them was in contact with the catalyst bed. Via an external heat supply the temperature of the catalyst fixed bed was adjusted to a value of ca. 380°C. The reaction conditions for the catalytic testing of each catalyst are summarized in the tables 2 to 5 below. The temperatures, which are explicitly mentioned in these tables and which differ from 380°C, i.e. 300, 320, 340, 345, 400 and 420, refer to the temperature of the oven surrounding the catalyst containing reactor. Each of the reactors, which were filled with a specific mixed oxide catalyst, was supplied in three tests with a feed gas having the composition N₂/O₂/C₃H₆/H₂ with a ratio of 70/14/8/8 at different ratios of catalyst mass to feed gas flow rate (w/F), also referred to as modified residence time. In the examples 3.1 and 3.2, where the catalysts according to the present invention were tested, the modified residence times were 0.48 g s ml⁻¹, 0.32 g s ml⁻¹, or 0.24 g s ml⁻¹. In the comparative examples 3.3 to 3.5 the modified residence time were 0.96 g s ml⁻¹, 0.64 g s ml⁻¹, and 0.48 g s ml⁻¹ (because of the doubled mass of the catalysts in the comparative examples) as well as 0.32 g s ml⁻¹. The results of the catalytic testing, such as the propylene conversion and the selectivity for the formation of acrolein, acrylic acid and CO + CO₂ are summarized in the tables 2 to 5. During the tests the temperature at the entry of the catalyst fixed bed were essentially kept constant at the temperatures given in the tables by adjusting temperature at the entry of the fixed catalyst fixed bed with an external heat source in case of temperature fluctuations. The reaction gas mixture leaving the reactor was conducted to a gas chromatograph of the type Agilent 7890B via a heated conduit.

The gas chromatograph was equipped with two test sample loops: one for permanent gases, in particular nitrogen, oxygen, carbon monoxide and carbon dioxide, and the other for hydrocarbons, in particular propylene, acrolein and acrylic acid. The first sample loop consisted of two serially connected separation columns of the type Haysep Q from Agilent and a final micropacked separation column of the type Molsieve 5A from Agilent. The first Haysep Q-separation column served for the separation of the permanent gases from the hydrocarbons and the second Haysep Q-separation column served for the fractionation of the permanent gases: carbon dioxide was eluted from the second Haysep Q-separation column as the first gas, and in the following the other gases were eluted together from the second Haysep Q-separation column and fed via a valve to the final separation column of the type Molsieve 5A, which served for the separation of the permanent gases N₂, O₂, and CO. Carbon dioxide and the permanent gases, which were eluted from the Molsieve 5A separation column were detected with a heat conducting detector. The second sample loop consisted of a capillary column of the type HF-FFAP from Agilent. The compounds propylene, acrolein and acrylic acid were eluted from this column and detected with a flame ionization detector. The gas chromatograph was calibrated with gas mixtures containing known concentrations of N₂, CO, CO₂, propylene and propane as well as a gas mixture of N₂ and O₂, and with standard solutions of propylene, acrolein and acrylic acid in methanol. After these calibrations the separation and the determination of the individual components of the reaction gas mixtures was performed in the individual catalysts tests. The conversion of propylene and the selectivity for the formation of acrolein, acrylic acid, as well as for carbon monoxide and carbon dioxide were calculated from the quantity of propylene used in the tests and the detected amounts of propylene, acrolein, and acrylic acid as well as carbon monoxide and carbon dioxide. These results are summarized in the tables 2 to 5 below.

**Table 2: Overview over the propylene conversion (n.d. = not determined)**

| **Run** | **Catalyst** | **T/°C** | **w/F = 0.96 g s mL⁻¹** | **w/F = 0.64 g s mL⁻¹** | **w/F = 0.48 g s mL⁻¹** | **w/F = 0.32 g s mL⁻¹** | **w/F = 0.24 g s mL⁻¹** |
|---|---|---|---|---|---|---|---|
| 1 | FSP-B_{1.5}Mo₁₂Co₅Fe_{1.8}Oₓ | 300 | n.d. | n.d. | 13.14 | 9.09 | 6.77 |
| 2 | FSP-B_{1.5}Mo₁₂Co₅Fe_{1.8}Oₓ | 320 | n.d. | n.d. | 88.20 | 84.34 | 82.09 |
| 3 | FSP-B_{1.5}Mo₁₂Co₅Fe_{1.8}Oₓ | 330 | n.d. | n.d. | 92.28 | 84.67 | 80.83 |
| 4 | FSP-B_{1.5}Mo₁₂Co₅Fe_{1.8}Oₓ | 340 | n.d. | n.d. | 89.47 | 86.32 | 86.89 |
| 5 | FSP-Bi₁Mo₁₂Co₈Fe₃Oₓ | 300 | n.d. | n.d. | 6.53 | 5.46 | 3.52 |
| 6 | FSP-Bi₁Mo₁₂Co₈Fe₃Oₓ | 320 | n.d. | n.d. | 21.27 | 16.75 | 13.14 |
| 7 | FSP-Bi₁Mo₁₂Co₈Fe₃Oₓ | 330 | n.d. | n.d. | 93.13 | 88.52 | 73.05 |
| 8 | FSP-Bi₁Mo₁₂Co₈Fe₃Oₓ | 340 | n.d. | n.d. | 92.75 | 89.21 | 87.63 |
| 9 | CP-Bi_{1.5}Mo₁₂Co₅Fe_{1.8}Oₓ | 345 | n.d. | n.d. | 14.20 | 5.69 | 6.83 |
| 10 | CP-Bi_{1.5}Mo₁₂Co₅Fe_{1.8}Oₓ | 380 | n.d. | n.d. | 42.45 | 34.71 | 27.80 |
| 11 | FSP-Bi₂Mo₃O₁₂ | 345 | 6.24 | 2.72 | 2.49 | 2.75 | n.d. |
| 12 | FSP-Bi₂Mo₃O₁₂ | 380 | 9.01 | 5.44 | 3.30 | 1.46 | n.d. |
| 13 | FSP-Bi₂Mo₃O₁₂ | 420 | 9.71 | 3.30 | 3.32 | 0 | n.d. |
| 14 | FSP-Bi₂MoO₆ | 345 | 45.72 | 29.72 | 21.13 | 13.08 | n.d. |
| 15 | FSP-Bi₂MoO₆ | 380 | 54.37 | 39.99 | 30.78 | 19.49 | n.d. |
| 16 | FSP-Bi₂MoO₆ | 400 | 49.55 | 39.26 | 30.23 | 18.95 | n.d. |
| 17 | FSP-Bi₂Mo₂O₉ | 345 | 13.61 | 9.67 | 7.14 | 5.71 | n.d. |
| 18 | FSP-Bi₂Mo₂O₉ | 380 | 31.75 | 22.13 | 15.52 | 8.96 | n.d. |
| 19 | FSP-Bi₂Mo₂O₉ | 400 | 40.47 | 28.47 | 21.79 | 14.42 | n.d. |

**Table 3: Overview over the acrolein selectivity (n.d. = not determined)**

| **Run** | **Catalyst** | **T/°C** | **w/F = 0.96 g s mL⁻¹** | **w/F = 0.64 g s mL⁻¹** | **w/F = 0.48 g s mL⁻¹** | **w/F = 0.32 g s mL⁻¹** | **w/F = 0.24 g s mL⁻¹** |
|---|---|---|---|---|---|---|---|
| 1 | FSP-B_{1.5}Mo₁₂Co₅Fe_{1.8}Oₓ | 300 | n.d. | n.d. | 81.40 | 83.69 | 84.16 |
| 2 | FSP-B_{1.5}Mo₁₂Co₅Fe_{1.8}Oₓ | 320 | n.d. | n.d. | 58.99 | 49.90 | 51.02 |
| 3 | FSP-B_{1.5}Mo₁₂Co₅Fe_{1.8}Oₓ | 330 | n.d. | n.d. | 37.39 | 60.74 | 75.30 |
| 4 | FSP-B_{1.5}Mo₁₂Co₅Fe_{1.8}Oₓ | 340 | n.d. | n.d. | 76.07 | 75.37 | 42.48 |
| 5 | FSP- Bi₁Mo₁₂Co₈Fe₃Oₓ | 300 | n.d. | n.d. | 90.04 | 90.13 | 100.00 |
| 6 | FSP- Bi₁Mo₁₂Co₈Fe₃Oₓ | 320 | n.d. | n.d. | 88.36 | 88.68 | 74.67 |
| 7 | FSP- Bi₁Mo₁₂Co₈Fe₃Oₓ | 330 | n.d. | n.d. | 68.27 | 78.10 | 73.05 |
| 8 | FSP- Bi₁Mo₁₂Co₈Fe₃Oₓ | 340 | n.d. | n.d. | 80.58 | 80.10 | 79.22 |
| 9 | CP-Bi_{1.5}Mo₁₂Co₅Fe_{1.8}Oₓ | 345 | n.d. | n.d. | 69.50 | 72.68 | 71.23 |
| 10 | CP-Bi_{1.5}Mo₁₂Co₅Fe_{1.8}Oₓ | 380 | n.d. | n.d. | 63.51 | 65.70 | 70.48 |
| 11 | FSP-Bi₂Mo₃O₁₂ | 345 | 85.37 | 88.13 | 88.20 | 90.90 | n.d. |
| 12 | FSP-Bi₂Mo₃O₁₂ | 380 | 93.12 | 94.32 | 94.32 | 95.59 | n.d. |
| 13 | FSP-Bi₂Mo₃O₁₂ | 420 | 84.47 | 95.64 | 96.40 | 0 | n.d. |
| 14 | FSP-Bi₂MoO₆ | 345 | 76.59 | 87.60 | 90.50 | 94.00 | n.d. |
| 15 | FSP-Bi₂MoO₆ | 380 | 81.12 | 90.76 | 92.92 | 94.93 | n.d. |
| 16 | FSP-Bi₂MoO₆ | 400 | 86.49 | 91.58 | 94.55 | 96.05 | n.d. |
| 17 | FSP-Bi₂Mo₂O₉ | 345 | 74.98 | 79.61 | 76.56 | 74.84 | n.d. |
| 18 | FSP-Bi₂Mo₂O₉ | 380 | 73.83 | 84.64 | 86.35 | 88.05 | n.d. |
| 19 | FSP-Bi₂Mo₂O₉ | 400 | 77.74 | 87.16 | 89.81 | 90.77 | n.d. |

**Table 4: Overview over the CO + CO₂ selectivity (n.d. = not determined)**

| **Run** | **Catalyst** | **T/°C** | **w/F = 0.96 g s mL⁻¹** | **w/F = 0.64 g s mL⁻¹** | **w/F = 0.48 g s mL⁻¹** | **w/F = 0.32 g s mL⁻¹** | **w/F = 0.24 g s mL⁻¹** |
|---|---|---|---|---|---|---|---|
| 1 | FSP-B_{1.5}Mo₁₂Co₅Fe_{1.8}Oₓ | 300 | n.d. | n.d. | 18.60 | 16.31 | 15.84 |
| 2 | FSP-B_{1.5}Mo₁₂Co₅Fe_{1.8}Oₓ | 320 | n.d. | n.d. | 9.93 | 4.94 | 5.02 |
| 3 | FSP-B_{1.5}Mo₁₂Co₅Fe_{1.8}Oₓ | 330 | n.d. | n.d. | 4.11 | 6.11 | 7.65 |
| 4 | FSP-B_{1.5}Mo₁₂Co₅Fe_{1.8}Oₓ | 340 | n.d. | n.d. | 8.63 | 8.46 | 5.04 |
| 5 | FSP-Bi₁Mo₁₂Co₈Fe₃Oₓ | 300 | n.d. | n.d. | 9.96 | 9.87 | 0 |
| 6 | FSP-Bi₁Mo₁₂Co₈Fe₃Oₓ | 320 | n.d. | n.d. | 11.64 | 11.32 | 9.19 |
| 7 | FSP-Bi₁Mo₁₂Co₈Fe₃Oₓ | 330 | n.d. | n.d. | 10.95 | 12.92 | 13.81 |
| 8 | FSP-Bi₁Mo₁₂Co₈Fe₃Oₓ | 340 | n.d. | n.d. | 16.00 | 16.39 | 17.42 |
| 9 | CP-Bi_{1.5}Mo₁₂Co₅Fe_{1.8}Oₓ | 345 | n.d. | n.d. | 29.80 | 26.71 | 28.18 |
| 10 | CP-Bi_{1.5}Mo₁₂Co₅Fe_{1.8}Oₓ | 380 | n.d. | n.d. | 27.70 | 20.18 | 19.05 |
| 11 | FSP-Bi₂Mo₃O₁₂ | 345 | 14.63 | 11.87 | 11.80 | 9.10 | n.d. |
| 12 | FSP-Bi₂Mo₃O₁₂ | 380 | 6.88 | 5.68 | 5.68 | 4.41 | n.d. |
| 13 | FSP-Bi₂Mo₃O₁₂ | 420 | 5.53 | 4.36 | 3.57 | 0 | n.d. |
| 14 | FSP-Bi₂MoO₆ | 345 | 19.69 | 9.92 | 7.81 | 6.00 | n.d. |
| 15 | FSP-Bi₂MoO₆ | 380 | 15.57 | 7.17 | 5.37 | 3.59 | n.d. |
| 16 | FSP-Bi₂MoO₆ | 400 | 12.36 | 5.13 | 4.00 | 2.87 | n.d. |
| 18 | FSP-Bi₂Mo₂O₉ | 345 | 25.02 | 20.39 | 23.44 | 25.16 | n.d. |
| 18 | FSP-Bi₂Mo₂O₉ | 380 | 24.52 | 14.97 | 13.65 | 11.95 | n.d. |
| 19 | FSP-Bi₂Mo₂O₉ | 400 | 20.81 | 11.35 | 9.90 | 9.23 | n.d. |

**Table 5: Overview over the acrylic acid selectivity (n.d. = not determined)**

| **Run** | **Catalyst** | **T/°C** | **w/F = 0.96 g s mL⁻¹** | **w/F = 0.64 g s mL⁻¹** | **w/F = 0.48 g s mL⁻¹** | **w/F = 0.32 g s mL⁻¹** | **w/F = 0.24 g s mL⁻¹** |
|---|---|---|---|---|---|---|---|
| 1 | FSP-Bi_{1.5}Mo₁₂Co₅Fe_{1.8}Oₓ | 300 | n.d. | n.d. | 0 | 0 | 0 |
| 2 | FSP-Bi_{1.5}Mo₁₂Co₅Fe_{1.8}Oₓ | 320 | n.d. | n.d. | 30.79 | 45.01 | 43.83 |
| 3 | FSP-Bi_{1.5}Mo₁₂Co₅Fe_{1.8}Oₓ | 330 | n.d. | n.d. | 58.44 | 32.98 | 16.86 |
| 4 | FSP-Bi_{1.5}Mo₁₂Co₅Fe_{1.8}Oₓ | 340 | n.d. | n.d. | 15.05 | 15.94 | 52.43 |
| 5 | FSP-Bi₁Mo₁₂Co₈Fe₃Oₓ | 300 | n.d. | n.d. | 0 | 0 | 0 |
| 6 | FSP-Bi₁Mo₁₂Co₈Fe₃Oₓ | 320 | n.d. | n.d. | 0 | 0 | 0 |
| 7 | FSP-Bi₁Mo₁₂Co₈Fe₃Oₓ | 330 | n.d. | n.d. | 20.60 | 8.77 | 12.93 |
| 8 | FSP-Bi₁Mo₁₂Co₈Fe₃Oₓ | 340 | n.d. | n.d. | 31.13 | 3.11 | 3.16 |
| 9 | CP-Bi_{1.5}Mo₁₂Co₅Fe_{1.8}Oₓ | 345 | n.d. | n.d. | 0 | 0 | 0 |
| 10 | CP-Bi_{1.5}Mo₁₂Co₅Fe_{1.8}Oₓ | 380 | n.d. | n.d. | 8.28 | 13.76 | 10.13 |
| 11 | FSP-Bi₂Mo₃O₁₂ | 345 | 0 | 0 | 0 | 0 | n.d. |
| 12 | FSP-Bi₂Mo₃O₁₂ | 380 | 0 | 0 | 0 | 0 | n.d. |
| 13 | FSP-Bi₂Mo₃O₁₂ | 420 | 0 | 0 | 0 | 0 | n.d. |
| 14 | FSP-Bi₂MoO₆ | 345 | 3.15 | 2.16 | 1.69 | 0 | n.d. |
| 15 | FSP-BbMoO₆ | 380 | 1.88 | 1.85 | 1.70 | 1.48 | n.d. |
| 16 | FSP-Bi₂MoO₆ | 400 | 9.47 | 3.11 | 1.45 | 1.08 | n.d. |
| 17 | FSP-Bi₂Mo₂O₉ | 345 | 0 | 0 | 0 | 0 | n.d. |
| 18 | FSP-Bi₂Mo₂O₉ | 380 | 1.06 | 0 | 0 | 0 | n.d. |
| 19 | FSP-Bi₂Mo₂O₉ | 400 | 0.90 | 1.10 | 0 | 0 | n.d. |

### Example 4: Determination of the phases with Raman spectroscopy

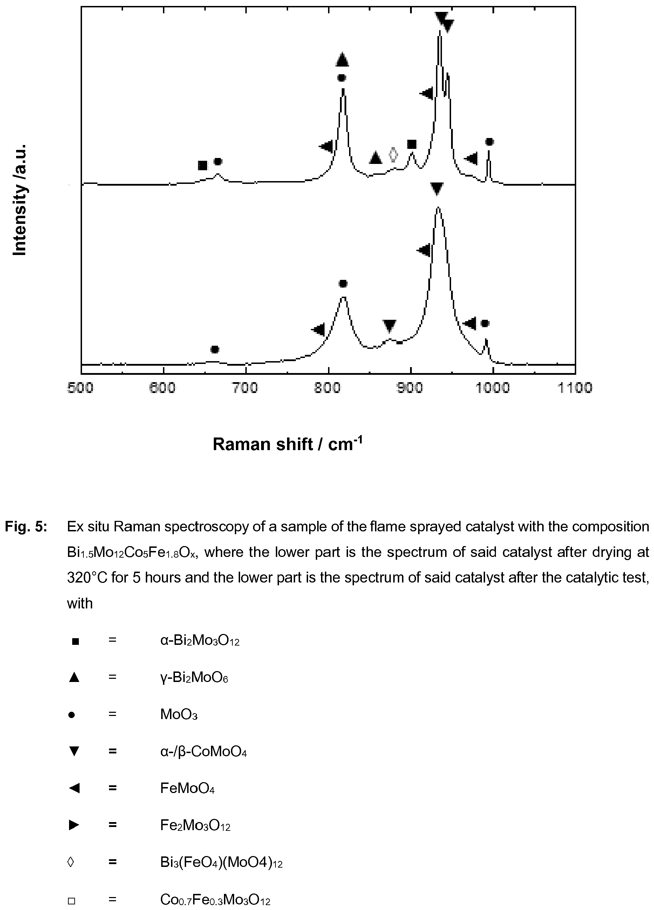

The phases of a sample of the catalyst with the composition Bi_{1.5}Mo₁₂Co₅Fe_{1.8}Oₓ of the present invention were determined using Raman spectroscopy. The samples were subjected to phase determinations after drying of the prepared catalyst at 320°C for 5 hours and after the catalytic testing of said catalyst. The apparatus for Raman spectroscopy was a Reishaw inVia Reflex spectrometer system of the firm Renishaw, which was equipped with a Research Grade Leica DM 2500 microscope. The samples were measured using a helium neon laser with a power of 1.7 mW at a wavelength of 623 nm. Typically, a spectral range of from 50 to 1300 cm⁻¹ was recorded. The recorded spectra is shown in figure 5.

### Example 5: Determination of the phases with X-ray diffraction

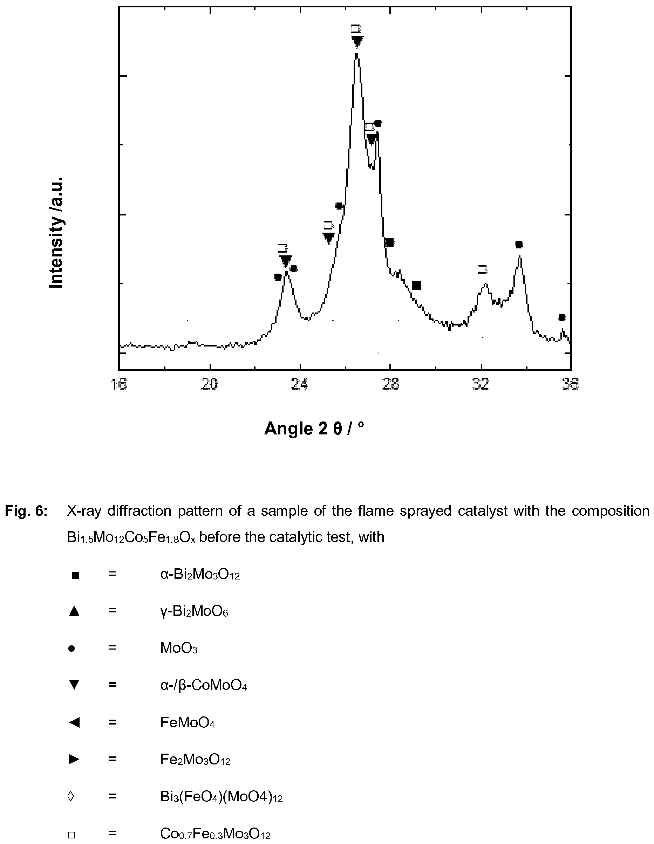

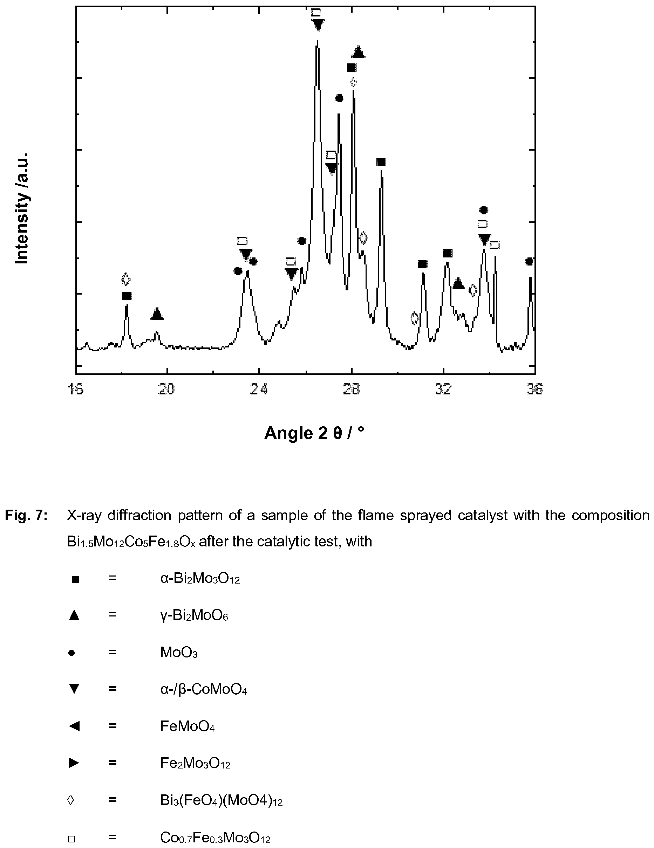

The phases of a sample of the catalyst with the composition Bi_{1.5}Mo₁₂Co₅Fe_{1.8}Oₓ according to the present invention were determined before and after the catalytic testing by X-ray diffraction using a Bruker D8 Advance diffractometer in the range 2θ = 8 - 80° (step size 0.016°) with the Cu Kα radiation (Ni-filter, 45 mA, 35 kV) in Bragg-Brentano geometry on rotating sample holders. The X-ray diffraction pattern of the catalyst before the catalytic testing is shown in figure 6, and the X-ray diffraction pattern of the catalyst before the catalytic testing is shown in figure 7.

### Example 6: Elementary analysis

The composition of the catalysts of the examples 1 and 2 was determined using optical emission spectroscopy with inductive coupled plasma (ICP-OES, type 720/725-ES by the firm Agilent). The plasma was produced by a 40 MHz high frequency generator, and argon was used as plasma gas. For the optical emission spectroscopy approximately 40 mg of the probe were suspended in a mixture of 6 mL concentrated hydrochloric acid, 2 mL concentrated nitric acid and 1 mL hydrogen peroxide, followed by treatment of the thus obtained mixture in a microwave at 600 watt for 45 minutes. The spectra of the catalysts were measured before and after their use in a catalyzed reaction.

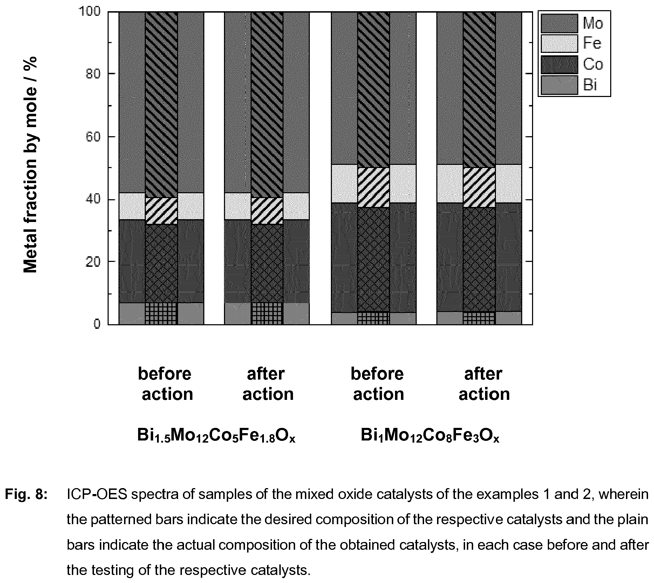

The ICP-OES spectra of samples of the catalysts of the examples 1 and 2 are shown in Figure 8. These spectra show that the process according to the present invention yields mixed oxide catalysts with each having a composition, which is almost identical to the desired composition of the catalysts to be prepared. Further, said ICP-OES spectra also show that the use of the flame sprayed catalysts in catalyzed reactions does not lead to a change of their composition.

### Example 7: Surface area determination of flame sprayed catalysts

The specific surface areas of the catalysts Bi₁Mo₁₂Mo₈Fe₃Oₓ and Bi_{1.5}Mo₁₂Co₅Fe_{1.8}Ox according to the present invention (FSP-Bi₁Mo₁₂Mo₈Fe₃Oₓ and FSP-Bi_{1.5}Mo₁₂Co₅Fe_{1.8}Ox) and of the catalysts Bi₂Mo₃Oₓ and Bi₂Mo₂Oₓ (FSP-Bi₂Mo₃Oₓ and FSP-Bi₂Mo₂Oₓ) which were all prepared by flame spray pyrolysis, and of the co-precipitated catalyst Bi_{1.5}Mo₁₂Co₅Fe_{1.8}Oₓ (CP-Bi_{1.5}Mo₁₂Co₅Fe_{1.8}Oₓ) were determined using the BET method. For this purpose 100 to 500 mg of the catalyst were dried in vacuum at 150°C for 5 hours. The BET measurements were carried out with a BELSORP-mini II of the firm Rubotherm GmbH, and helium was used as flushing gas. After drying, the catalyst samples were evacuated and cooled to 77 K with liquid nitrogen. The partial pressure of the adsorption gas nitrogen was increased step-wise, and a standard adsorption isotherm in the range p/po of from 0 to 1 was recorded. In the following, the desorption isotherm was recorded. The determination of the BET surface area was carried out using the BET theory in the range p/po of from 0.05 to 0.3. The measurements were done with a dried catalyst, i.e. before the catalytic testing, and after catalytic testing. The results are summarized in the table 6 below:

**Table 6: Overview over measured specific surface areas A_{BET} (n.d. = not determined)**

| | **Specific surface area A_{BET} / m² g⁻¹** | |
|---|---|---|
| **Catalyst** | **Dried** | **After catalytic testing** |
| FSP-Bi₁Mo₁₂Mo₈Fe₃Oₓ | 63 | 9 |
| FSP-Bi_{1.5}Mo₁₂Co₅Fe_{1.8}Oₓ | 43 | 17 |
| CP-Bi_{1.5}MO₁₂CO₅Fe_{1.8}Oₓ | 7 | 4 |
| FSP-Bi₂Mo₃Oₓ | 19 | n.d. |
| FSP-Bi₂Mo₂Oₓ | 18 | n.d. |

### Example 8: Analysis of the catalyst surfaces with TEM

The surface of a sample of the mixed oxide catalyst with the composition Bi₁Mo₁₂Co₈Fe₃Oₓ of example 2 was investigated using a transmission electron microscope (TEM) before and after the use of said catalyst in a catalyzed reaction.

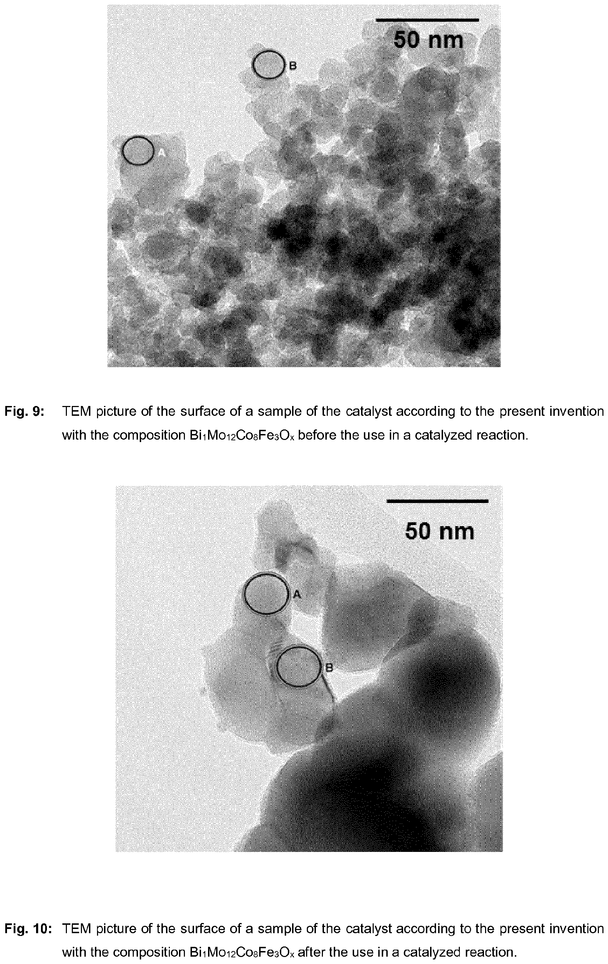

The TEM pictures are shown in figures 9 and 10. These pictures show that the catalyst has a particle diameter of not more than 50 nm before the use in a catalyzed reaction and a particle diameter of not more than 100 nm after the use in a catalyzed reaction.

## Claims

1. Process for the preparation of a mixed oxide catalyst, comprising bismuth, cobalt, iron and molybdenum, comprising the steps
a) providing a solution, suspension and/or slurry comprising precursors of bismuth, cobalt, iron and molybdenum in an organic solvent,
b) converting the solution, suspension and/or slurry of step a) to an aerosol,
c) introducing the aerosol of step b) into a pyrolysis zone,
d) performing a flame spray pyrolysis to give mixed oxide particles, and
e) collecting the mixed oxide particles obtained in step d).

2. Process according to Claim 1, wherein the step e) is performed by quenching of the mixed oxide particles on a cold surface.

3. Process according to Claim 1 or 2, wherein the precursors are organic compounds of bismuth, cobalt, iron and molybdenum.

4. Process according to any of Claims 1 to 3, wherein the precursors are salts and/or complexes of bismuth, molybdenum, cobalt and iron with an organic acid, organic alcohol and/or a chelating agent.

5. Process according to Claim 4, wherein the organic acid is a linear or branched C₁ to C₁₀ carboxylic acid.

6. Process according to Claim 4 or 5, wherein the organic acid is 2-ethylhexanoic acid.

7. Process according to Claim 4, wherein the chelating agent is a C₁ to C₁₀ compound with a hydroxyl, carbonyl, amino and/or amide group.

8. Process according to Claim 4 or 7, wherein the chelating agent is acetylaceton.

9. Process according to any of Claims 1 to 8, further comprising providing a solution, suspension and/or slurry comprising a precursor of a carrier material in step a), converting the same to an aerosol in step b), introducing the aerosol into the pyrolysis zone in step c), performing a flame spray pyrolysis to give supported mixed oxide particles in step d), and collecting the supported mixed oxide particles in step e).

10. Process according to any of Claims 1 to 9, further comprising the steps
f) applying the mixed oxide particles or supported mixed oxide particles obtained in step e) to a carrier to yield a supported catalyst,
or
f') shaping the mixed oxide particles or supported mixed oxide particles obtained in step e) to yield a solid catalyst,
and
g) drying and/or calcining the supported or solid catalyst obtained in step f) or f').

11. Mixed oxide catalyst, comprising bismuth, cobalt, iron and molybdenum, wherein said catalyst comprises a cobalt-molybdenum phase, an iron-molybdenum phase and/or a mixture of these phases.

12. Mixed oxide catalyst according to Claim 11, wherein the catalyst corresponds to the general formula
MoₐBi_{b}Co_{c}Fe_{d}NiₑX_{f}X'_{g}X"ₕX'"ᵢX""ⱼOₓ (I)
with
X = W and/or P,
X' = Li, Na, K, Rb, Cs, Mg, Ca, Ba and/or Sr,
X" = Ce, Mn, Cr and/or V,
X"' = Nb, Se, Te, Sm, Gd, La, Y, Pd, Pt, Ru and/or Au,
X"" = Si, Al, Ti and/or Zr,
and
a = 12,
b = 1 to 4,
c = 4 to 10,
d = 1 to 4,
e = 0 to 4,
f = 0 to 5,
g = 0 to 2,
h = 0 to 5,
i = 0 to 2,
j = 0 to 800,
and
x = a number, which depends on the valence and amount of the elements different from oxygen.

13. Mixed oxide catalyst according to Claim 11 or 12, wherein the catalyst is a flame sprayed mixed oxide catalyst.

14. Mixed oxide catalyst according to any of claims 11 to 13, wherein the mixed oxide catalyst has a particle diameter of not more than 100 nm.

15. Use of a mixed oxide catalyst obtained and/or obtainable by a process according to any of Claims 1 to 10 and/or of a mixed oxide catalyst according to any of Claims 11 to 14 in the partial gas phase oxidation and/or ammoxidation of olefins and/or alcohols.
